# EUROPÄISCHE PATENTSCHRIFT

(11) **EP 2 986 228 B1**
(45) Veröffentlichungstag und Bekanntmachung des Hinweises auf die Patenterteilung: **14.11.2018**
(21) Anmeldenummer: 14721787.1
(22) Anmeldetag: 16.04.2014
(51) Int. Cl.: A61B 10/04, A61B 10/00

(54) **VORRICHTUNG ZUR PROBENNAHME**
DEVICE FOR SAMPLING
DISPOSITIF DE PRÉLÈVEMENT D'ÉCHANTILLONS

(30) Priorität: 16.04.2013 AT 3112013
(43) Veröffentlichungstag der Anmeldung: 24.02.2016
(73) Patentinhaber: Mitterer, Stephan, 2340 Mödling (AT)
(72) Erfinder: Mitterer, Stephan, 2340 Mödling (AT)
(74) Vertreter: KLIMENT & HENHAPEL
(86) Internationale Anmeldenummer: PCT/EP2014/057794
(87) Internationale Veröffentlichungsnummer: WO 2014/170393

(56) Entgegenhaltungen:
- GB-A- 1 547 328
- US-A- 5 192 290
- US-A- 5 599 298
- US-B1- 6 576 429

## Beschreibung

Die vorliegende Erfindung bezieht sich auf eine Probennahmevorrichtung zum Einführen in den Ösophagus für die Entnahme von Proben zur medizinischen Diagnostik, welche einen Schlauch mit einem in den Ösophagus einführbaren Endbereich umfasst, sowie einem am Endbereich befestigten Probennehmer, wobei der Schlauch einen im einführbaren Endbereich axial geschlossenen Kanal mit einer radialen Schlauchwandöffnung aufweist, gemäß dem Oberbegriff des Anspruchs 1.

Die Überlebensrate bei Speiseröhrenkrebs liegt nach 5 Jahren bei unter 20%. Handelt es sich aber um die Vorstufe "Barrett Ösophagus", kann er relativ gut therapiert werden. Die derzeit übliche Diagnosemethode ist eine Gastroskopie, bei welcher Gewebeproben aus der Mukosa (Speiseröhreninnenwand) der Speiseröhre entnommen werden. Dabei werden ein Schlauch mit einer Kamera und eine Biopsiezange in die Speiseröhre (Ösophagus) des Patienten eingeführt und anschließend entnimmt der Arzt an bestimmten Stellen mit der Zange Proben der Mukosa, wobei er mithilfe der Kamera den Vorgang steuern kann.

Da die Proben nicht lückenlos aus der Oberfläche der Mukosa entnommen werden können, passiert es zwangsläufig, dass Biopsien an Stellen genommen werden, an denen noch keine Gewebeveränderungen vorliegen, während an anderen Stellen, an denen keine Proben entnommen werden, bereits Entartungen vorliegen könnten. Dies führt dazu, dass die Diagnosemethode nicht die gewünschte Zuverlässigkeit aufweist, eine frühzeitige Erkennung aber, wie eingangs erwähnt, für einen Behandlungserfolg von großer Bedeutung ist.

Die Zeit, die für eine Gastroskopie mit Probenentnahme mithilfe der Biopsiezange an verschiedenen Stellen des Zwölffingerdarms, des Magens sowie der Speiseröhre bei genauerer Durchführung, beispielsweise nach dem Chandrasoma-Protokoll, benötigt wird, liegt außerdem bei bis zu 45 Minuten. Hierbei befindet sich der Patient meist unter Narkose, insbesondere bei längeren Untersuchungen. Je länger sich der Patient im Tiefschlaf befindet, umso größer ist die Gefahr von Komplikationen während und auch nach der Untersuchung. Ein weiterer Nachteil besteht darin, dass es bei der Gewebeentnahme zu Perforationen der Mukosa kommen kann.

Daher wurde vorgeschlagen, einen Schlauch mit einem aufblasbaren Ballon in die Speiseröhre einzuführen. Der Ballon befindet sich am Ende des Schlauches und ist von einem Netz umgeben. Hat der Ballon nun den Magen erreicht, so wird er über den Schlauch aufgepumpt und anschließend unter Widerstand durch die Speiseröhre herausgezogen. Dabei bleiben reibungsbedingt Gewebeproben der Speiseröhre am Netz hängen. Diese Gewebeproben können anschließend aus dem Netz extrahiert und im Labor weiter untersucht werden.

Es hat sich jedoch herausgestellt, dass das Netz kein gut geeignetes Mittel ist, um Proben zu entnehmen, denn einige Gewebeproben werden entweder nicht entnommen, oder gehen beim weiteren Herausziehen wieder verloren. Des Weiteren erschwert der am Schlauchende angeordnete Ballon das Einführen in den Ösophagus, insbesondere ist ein Einführen in den Arbeitskanal eines Endoskops unmöglich, weshalb der Arzt den Vorgang nicht visuell mitverfolgen kann. Dies hat den Nachteil, dass der Arzt nicht sieht, wie stark der Ballon aufgepumpt ist. Ist er zu wenig aufgepumpt, so werden zu wenig Gewebeproben entnommen; ist er zu stark aufgepumpt, so besteht die Gefahr einer Verletzung der Mukosa. Da der Speiseröhrenkanal nicht überall den gleichen Durchmesser aufweist ist der Ballon zwangsweise nie überall optimal aufgepumpt. Darüber hinaus bleiben alle auftretenden Komplikationen dem Arzt verborgen.

Weitere Probennahmevorrichtungen wurden in der US 5,599,298, die den nächstliegenden Stand der Technik darstellt, der GB 1 547 328, der US 5,192,290 sowie der US 6,576,429 beschrieben.

Es ist daher das Ziel der Erfindung diese Nachteile zu vermeiden und eine Probennahmevorrichtung für die Entnahme von Proben aus dem Ösophagus zur medizinischen Diagnostik vorzuschlagen, mit der Proben flächendeckend der Speiseröhre entnommen werden können, ohne dass dabei die Mukosa verletzt wird und darüber hinaus die dafür benötigte Zeit gegenüber einer Entnahme mit einer Biopsiezange verringert wird. Eine weitere Aufgabe besteht darin, die Vorrichtung in Größe und Beschaffenheit derart zu gestalten, dass sie in ein Endoskop eingebracht werden kann und somit dem Arzt ermöglicht, die Untersuchung visuell mitzuverfolgen.

Diese Aufgaben werden durch eine Probennahmevorrichtung mit den Merkmalen von Anspruch 1 gelöst. Anspruch 1 bezieht sich dabei auf eine Probennahmevorrichtung zum Einführen in den Ösophagus für die Entnahme von Proben zur medizinischen Diagnostik, welche einen Schlauch mit einem in den Ösophagus einführbaren Endbereich umfasst, sowie einem am Endbereich befestigten Probennehmer, wobei der Schlauch einen im einführbaren Endbereich axial geschlossenen Kanal mit einer radialen Schlauchwandöffnung aufweist und der Schlauch entlang eines axialen Abschnittes seines Endbereiches von einer die Schlauchwandöffnung bedeckenden und aufblasbaren Membran umspannt ist. Dabei ist es erfindungsgemäß vorgesehen, dass die Membran in zwei beidseits der Schlauchwandöffnung angeordneten Membranbefestigungsbereichen am Schlauch befestigt und von einer schwammartigen Umhüllung als Probennehmer umgeben ist, wobei die schwammartige Umhüllung in zwei beidseits der Schlauchwandöffnung angeordneten Umhüllungsbefestigungsbereichen am Schlauch befestigt ist, und die Umhüllungsbefestigungsbereiche in axialer Richtung weiter voneinander entfernt sind als die Membranbefestigungsbereiche.

Eine derart gestaltete Vorrichtung ist durch die schwammartige Umhüllung gut geeignet, Proben flächendeckend zu entnehmen. Dabei ändert sich diese Eigenschaft durch stärkeres Aufblasen der Membran nicht. Die Oberfläche der schwammartigen Umhüllung weist einen stark abrasiven Charakter auf, wodurch die Probenentnahme begünstigt wird. Bei Aufblasen der Membran wird sich rasch eine annähernd kugelförmige Konfiguration einstellen, die von der schwammartigen Umhüllung umgeben ist. Die schwammartige Struktur stellt sicher, dass Zellen, die beim Beginn der Probennahme etwa am magenseitigen Ausgang der Speiseröhre entnommen werden, während des weiteren Bewegungsweges des Probennehmers in Richtung des Speiseröhreneinganges von nachfolgend abgeschabten Zellen in das Schwamminnere verdrängt werden und daher nicht vom Probennehmer abfallen. Zudem ist die Ausdehnung des Probennehmers leicht variierbar, ohne dass die Qualität der Probennahme beeinträchtigt wird. Die erfindungsgemäße Probennahmevorrichtung kann daher auf eine unterschiedliche Größe und Beschaffenheit des Speiseröhrenquerschnittes leicht angepasst werden, insbesondere kann auch die Elastizität der Speiseröhre selbst ausgenutzt werden, um durch entsprechend starkes Aufblasen der Membran die Speiseröhre geringfügig zu dehnen und eine gute Anlage der schwammartigen Umhüllung an der Speiseröhrenwand zu bewerkstelligen.

Im unaufgeblasenen Zustand liegen sowohl die Membran als auch die schwammartige Umhüllung hingegen eng am Schlauch an, wodurch sich im unaufgeblasenen Zustand ein schlanker Körper ergibt. Dies ermöglicht das Einführen der Vorrichtung in ein Endoskop. Der dadurch gewonnene Nutzen ist eine visuelle Überprüfung des Gewebeentnahmevorganges durch den Arzt über die Kamera des Endoskops. Aber auch der Gebrauch ohne Endoskop ist möglich.

Nachdem die Probennahmevorrichtung in den Magen eingebracht wurde, pumpt sie der Arzt etwa mithilfe einer luftgefüllten Spritze auf, um anschließend das Endoskop mitsamt der Probennahmevorrichtung durch den Ösophagus herauszuziehen. Dabei verfolgt er den Vorgang über die Kamera und verändert die Aufblasgröße der Membran und der schwammartigen Umhüllung je nach lokalen Gegebenheiten des Ösophagus. Ist dieser schmäler, so verkleinert der Arzt den Durchmesser der aufgeblasenen Membran; ist er breiter, so pumpt er sie stärker auf. Die Membran bekommt dadurch die optimale Größe für eine flächendeckende Gewebeprobenentnahme ohne jedoch die Mukosa zu verletzen. Treten Komplikationen auf, so bemerkt dies der Arzt und er ist imstande unverzüglich zu reagieren.

Die Zeit der Untersuchung wird im Vergleich mit einer Gewebeentnahme mittels Biopsiezange deutlich verkürzt, was die Risiken für den Patienten senkt.

In der Regel werden die schwammartige Umhüllung und die Membran nicht dieselbe Elastizität aufweisen. Um dennoch eine leichte Aufblasbarkeit der Membran und der sie umgebenden, schwammartigen Umhüllung zu erreichen wird erfindungsgemäß vorgeschlagen, dass die schwammartige Umhüllung in zwei beidseits der

Schlauchwandöffnung angeordneten Umhüllungsbefestigungsbereichen am Schlauch befestigt ist, wobei die Umhüllungsbefestigungsbereiche in axialer Richtung weiter voneinander entfernt sind als die Membranbefestigungsbereiche. Die geringere Dehnbarkeit pro Längeneinheit der schwammartigen Umhüllung wird somit durch Bereitstellung einer größeren Länge ausgeglichen.

Eine weitere Ausführungsvariante ist dergestalt, dass die schwammartige Umhüllung in ihren Umhüllungsbefestigungsbereichen mit jeweils zumindest einem Befestigungsring am Schlauch befestigt ist. Dieser stellt ein einfaches und effektives Befestigungsinstrument dar. Die Membran kann mit dem Schlauch verklebt und/oder mit einer Schnur und dergleichen befestigt sein.

Eine weitere Ausführungsvariante zeichnet sich dadurch aus, dass die Umhüllungsbefestigungsbereiche von einem Gummiüberzug umschlossen sind, weshalb die Mukosa vor Verletzungen geschützt wird, insbesondere durch umklappende Abschnitte der schwammartigen Umhüllung außerhalb der Befestigungsbereiche.

In einer weiteren Ausführungsvariante ist der Schlauch an seinem einführbaren Endbereich mit einem konisch zulaufenden Führungsfortsatz axial geschlossen, welcher das Ende des Schlauches bildet. Dadurch entsteht eine für das Einführen gut geeignete Stabilität der Probennahmevorrichtung, sowie aufgrund der Flexibilität des Führungsfortsatzes ein Schutz für die Mageninnenwand bei ihrem Kontakt mit dem Gerät.

Falls die Steifheit des Schlauches für ein leichtes Einführen in den Endoskopieschlauch bzw. die Speiseröhre unzureichend ist, wäre auch die Verwendung eines Versteifungsdrahtes denkbar. Hierzu wird vorgeschlagen, dass der Schlauch zweilumig ausgeführt ist und in einem der beiden Kanäle ein Versteifungsdraht verläuft.

Die Erfindung wird im Folgenden anhand von Ausführungsbeispielen mithilfe der beiliegenden Zeichnungen näher erläutert. Hierbei zeigen:
Fig. 1 eine Schnittansicht eines Teils eines menschlichen Körpers, wobei Mund, Rachen, Ösophagus, Magen und eine Ausführungsvariante einer erfindungsgemäßen Probennahmevorrichtung im unaufgeblasenen Zustand skizziert sind,
Fig. 2 eine weitere Schnittansicht wie in Fig. 1, wobei sich die Probennahmevorrichtung im aufgeblasenen Zustand befindet,
Fig. 3 eine weitere Schnittansicht wie in Fig. 1, wobei sich die Probennahmevorrichtung im aufgeblasenen Zustand befindet und der Probennahmevorgang in der Speiseröhre begonnen wurde,
Fig. 4 eine Schnittansicht eines Endbereiches einer Probennahmevorrichtung im unaufgeblasenen Zustand,
Fig. 5 eine Schnittansicht eines Endbereiches einer Probennahmevorrichtung im aufgeblasenen Zustand,
Fig. 6 eine Schnittansicht eines Endbereiches einer Probennahmevorrichtung im aufgeblasenen Zustand mit eingezeichneten Schnittebenen A-A und B-B,
Fig. 7 den Schnitt entlang der Schnittebene A-A der Fig. 6, und die
Fig. 8 den Schnitt entlang der Schnittebene B-B der Fig. 6.

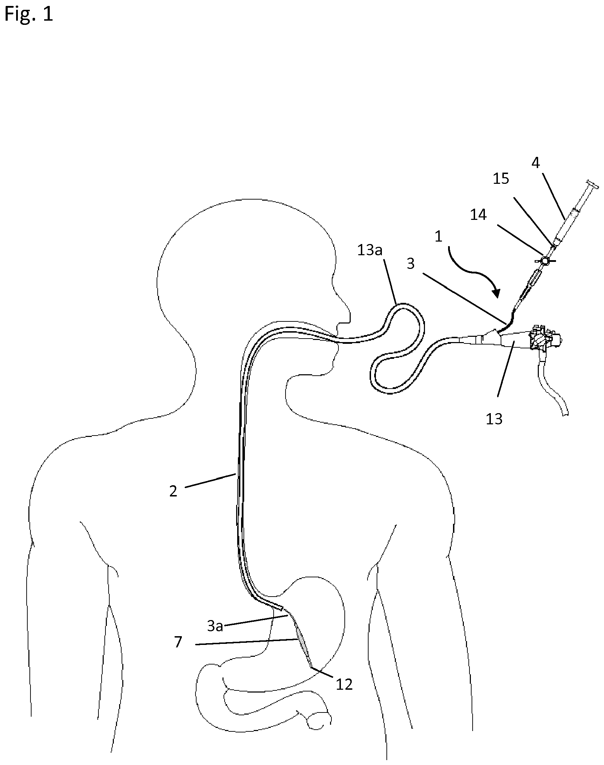

In der Fig. 1 ist ein Teil eines menschlichen Körpers im Bereich der Speiseröhre 2 (Ösophagus) angedeutet. Ein Endoskopieschlauch 13a eines Endoskop 13 ist in Mund, Rachen, Speiseröhre 2 und Magen eingebracht. In den Arbeitskanal des Endoskopieschlauches 13a wurde eine erfindungsgemäße Probennahmevorrichtung 1 eingeführt, die mit ihrem Endbereich 3a den Bereich des Magens erreicht hat. Ein Führungsfortsatz 12 erleichtert dabei das Einführen der Probennahmevorrichtung 1 in den Arbeitskanal des Endoskopieschlauches 13a.

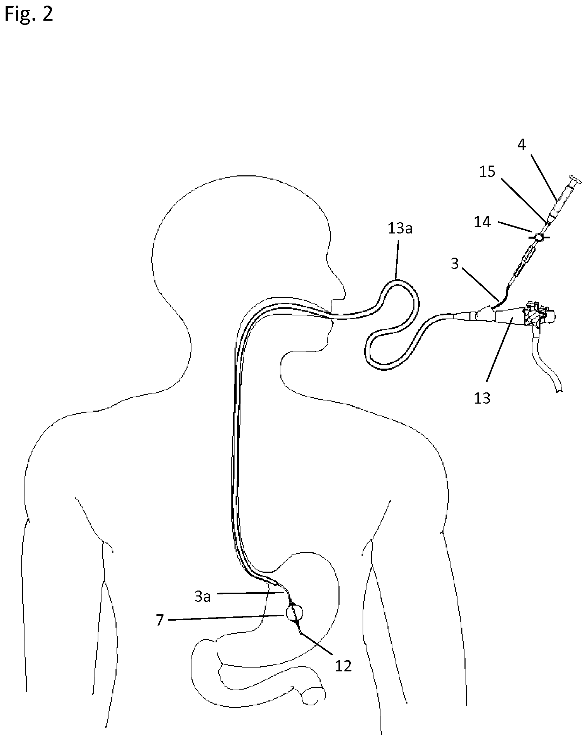

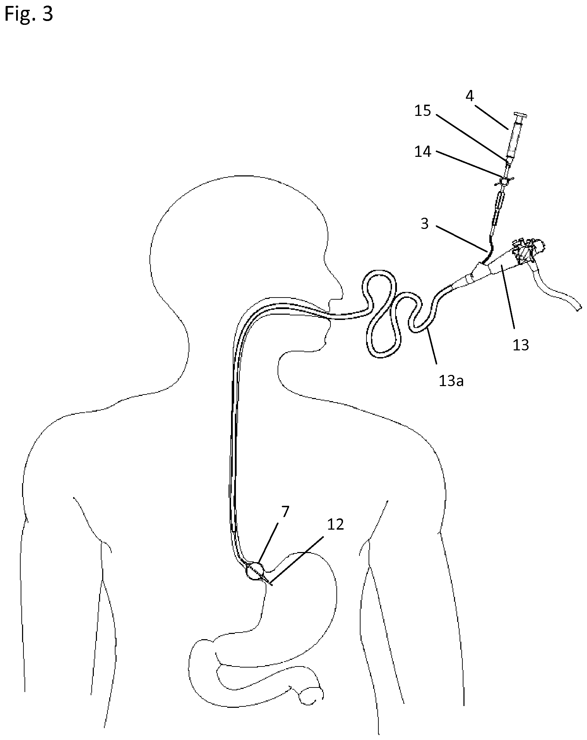

Die Probennahmevorrichtung 1 umfasst im Wesentlichen einen Schlauch 3 und einen Probennehmer in seinem Endbereich 3a, der in weiterer Folge noch genauer beschrieben wird. Jenes Ende des Schlauches 3, das außerhalb des Körpers des Patienten verbleibt, kann über eine Luer(-Lock)-Verbindung 15 an einer Spritze 4 befestigt werden, mit der ein gasförmiges Fluid in den Schlauch 3 gepresst werden kann. Ein Ventil 14 verhindert dabei ein Rückströmen des eingebrachten Fluids. Das gasförmige Fluid bewirkt dabei ein Aufblasen des Probennehmers, wie es in der Fig. 2 gezeigt ist. Durch vorsichtigen, gleichzeitigen Zug am Endoskopieschlauch 13a des Endoskops 13 und am Schlauch 3 der Probennahmevorrichtung 1 bewegt sich der aufgeblasene Probennehmer in Richtung der Speiseröhre, tritt in sie ein (siehe Fig. 3) und bewegt sich durch die gesamte Speiseröhre unter abrasivem Kontakt zur Speiseröhreninnenwand. Der abrasive Druck des Probennehmers an der Speiseröhreninnenwand kann dabei durch den Befüllungsdruck des Probennehmers gesteuert werden, die durch die Spritze 4 festgelegt werden kann. Das Ventil 14 entlastet dabei den Arzt, da der Druck im Probennehmer nicht permanent aktiv aufrecht erhalten werden muss. Da andererseits Verengungen der Speiseröhre Druck auf den Probennehmer ausüben, der das gasförmige Fluid in Richtung der Spritze 4 presst, kann der Arzt bei geöffnetem Ventil 14 Verengungen der Speiseröhre über den Kolbendruck der Spritze 4 feststellen. Bei Verengungen der Speiseröhre 2 kann der Befüllungsdruck durch geringfügiges Aufziehen der Spritze 4 wieder reduziert werden, um die Verengung leichter passieren zu können. Bei zu geringem Kontakt zur Speiseröhreninnenwand kann hingegen der Befüllungsdruck mithilfe der Spritze 4 erhöht werden, um eine zuverlässige Probennahme zu gewährleisten. Nach Durchqueren der Speiseröhre 2 wird der Probennehmer vom Schlauch 3 abgetrennt und in ein Konservierungsmittel, etwa Formalin, gelegt, um die Proben bis zur weiteren Analyse zu sichern. Die Proben können etwa zentrifugiert und mithilfe eines Mikroskops zytologisch untersucht werden, um etwa Hinweise auf eine Krebserkrankung zu finden. Bei negativem Befund kann eine aufwändige Biopsie in weiterer Folge unterbleiben.

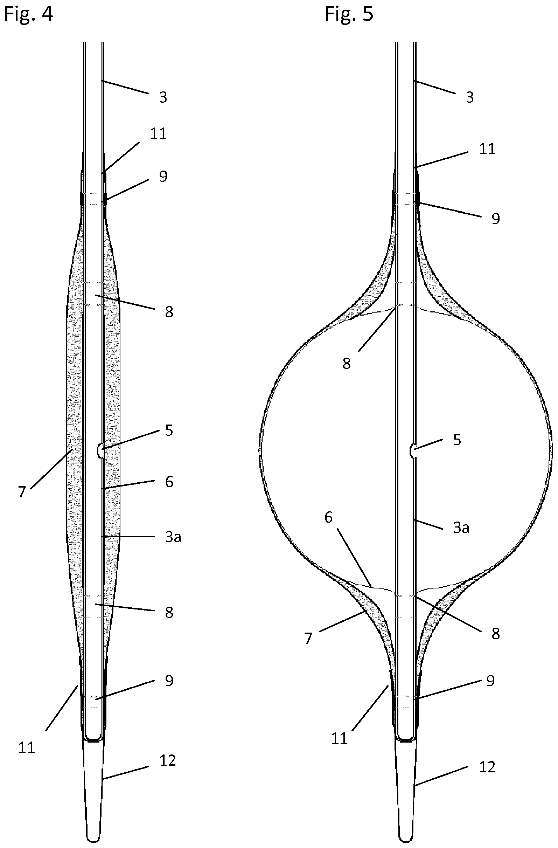

Anhand der Fig. 4-8 wird in weiterer Folge der Probennehmer genauer beschrieben. Der Probennehmer ist am einführbaren Endbereich 3a des Schlauches 3 angeordnet und umfasst eine aufblasbare Membran 6 und eine schwammartige Umhüllung 7. Das Schlauchende ist im einführbaren Endbereich 3a von einem konisch zulaufenden Führungsfortsatz 12 luftdicht verschlossen und mit einer radialen Schlauchwandöffnung 5 versehen. Die Membran 6 erstreckt sich entlang eines axialen Abschnittes des Endbereiches 3a und liegt im unaufgeblasenen Zustand eng am Schlauch 3 an, wobei sie den entsprechenden axialen Abschnitt des Endbereiches 3a umspannt und die Schlauchwandöffnung 5 bedeckt (siehe Fig. 4). Die Membran 6 ist dabei in zwei beidseits der Schlauchwandöffnung 5 angeordneten Membranbefestigungsbereichen 8 am Schlauch 3 befestigt und von einer eng anliegenden, schwammartigen Umhüllung 7 umgeben, die in zwei beidseits der Schlauchwandöffnung 5 angeordneten Umhüllungsbefestigungsbereichen 9 am Schlauch 3 befestigt ist, wobei die Umhüllungsbefestigungsbereiche 9 in axialer Richtung weiter voneinander entfernt sind als die Membranbefestigungsbereiche 8 (siehe Fig. 4-6). Auf diese Weise kann eine geringere Elastizität der schwammartigen Umhüllung 7 ausgeglichen werden.

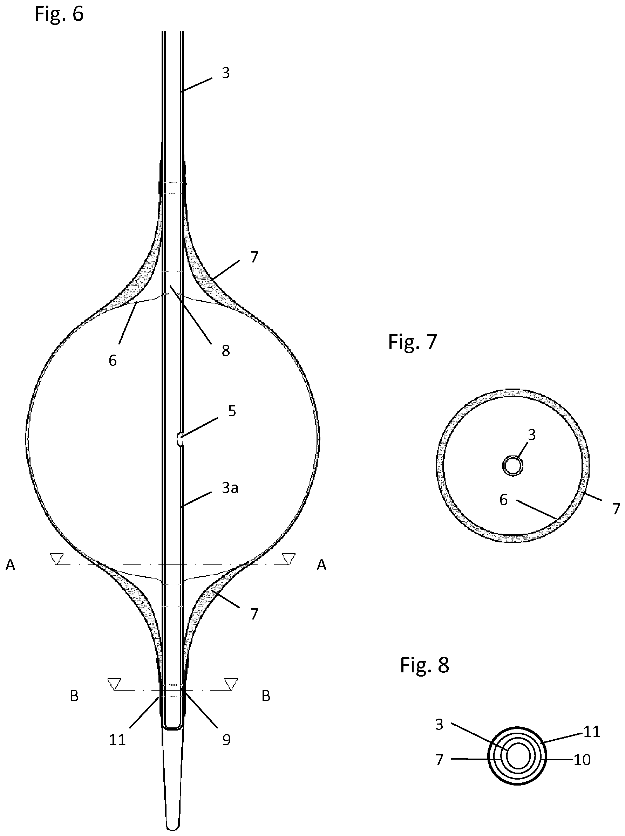

Die Membran 6 kann in ihren Membranbefestigungsbereichen 8 mit jeweils zumindest einem Faden oder einer Schnur am Schlauch 3 befestigt sein, oder auch mit dem Schlauch 3 verklebt oder angeformt werden. Des Weiteren kann die schwammartige Umhüllung 7 in ihren Umhüllungsbefestigungsbereichen 9 mit jeweils zumindest einem Befestigungsring 10 am Schlauch 3 befestigt sein, wie etwa in der Schnittansicht gemäß der Fig. 8 entlang der Schnittebene B-B ersichtlich ist. In der Fig. 8 sind dabei der innere Schlauch 3 zu sehen, der von der schwammartigen Umhüllung 7 umgeben ist. Die schwammartige Umhüllung 7 wird am Schlauch 3 durch den Befestigungsring 10 befestigt, dessen Außenkontur in der Fig. 8 eine dickere Kreislinie aufweist, die einen Gummiüberzug 11 darstellt, der den gesamten Aufbau umgibt. In der Fig. 7 ist eine Schnittansicht entlang der Schnittebene A-A ersichtlich, in der der Schlauch 3 sowie die schwammartige Umhüllung 7 sichtbar sind, wobei an der Innenseite der schwammartigen Umhüllung 7 die Membran 6 anliegt.

Mithilfe der Spritze 4 kann ein gasförmiges Fluid, etwa Luft, durch den Schlauch 3 in den Endbereich 3a gepresst werden, wo das gasförmige Fluid durch die Schlauchwandöffnung 5 austritt und die Membran 6 aufbläst. Der Probennehmer wechselt dabei von der Konfiguration gemäß Fig. 4 in eine Konfiguration gemäß der Fig. 5. Dabei wird auch die schwammartige Umhüllung 7, die an der Membran 6 anliegt, aufgeweitet. Es bildet sich annähernd eine Kugelform, deren äußere Oberfläche von schwammartigem Material gebildet wird und deren Durchmesser durch die Menge an eingeblasenem Fluid reguliert werden kann, die wiederum mithilfe der Spritze 4 gesteuert werden kann. Die schwammartige Umhüllung 7 ermöglicht eine gute Probenaufnahme und -rückhaltung während des gesamten Vorganges der Probennahme. Im Unterschied zur Biopsie können jedoch sehr rasch Proben aus der gesamten Speiseröhreninnenfläche gewonnen werden. Erst bei positivem Befund empfiehlt sich eine zeitaufwändigere und risikobehaftete Biopsie zur Lokalisierung der verdächtigen Stellen.

Ein besonderer Vorteil der erfindungsgemäßen Vorrichtung besteht darin, dass sie in den Arbeitskanal eines Endoskopieschlauches 13a eingebracht werden kann und somit dem Arzt ermöglicht, die Untersuchung visuell mitzuverfolgen. Durch die eng anliegende Membran 6 und die eng anliegende schwammartige Umhüllung 7 können vergleichsweise geringe Baugrößen mit Querschnittsdurchmesser unter 2.5mm erreicht werden. Die strukturelle Stabilität während des Einführens wird dem Probennehmer dabei durch den Schlauch 3 verliehen, an dem die Membran 6 und die schwammartige Umhüllung 7 anliegen. Falls die Steifheit des Schlauches 3 für ein leichtes Einführen in den Endoskopieschlauch 13a bzw. die Speiseröhre 2 unzureichend ist, wäre auch die Verwendung eines Versteifungsdrahtes denkbar, der innerhalb eines zweilumigen Schlauches 3 verläuft. In diesem Fall wäre es etwa möglich, den Versteifungsdraht in einem eigenen Kanal zu führen, sodass der Versteifungsdraht im eingeführten Endbereich 3a aus der Probennahmevorrichtung 1 austreten kann. Der Versteifungsdraht kann bei einer solchen Ausführungform vorgeschoben werden und als Führung für den nachgeschobenen Schlauch 3 dienen. Diese Ausführungsform eignet sich insbesondere auch bei Verwendungen ohne Endoskop, bei denen die erfindungsgemäße Probennahmevorrichtung 1 auch ohne endoskopische Unterstützung direkt in die Speiseröhre 2 eingeschoben wird.

### BEZUGSZEICHENLISTE

- 1: Probennahmevorrichtung
- 2: Speiseröhre (Ösophagus)
- 3: Schlauch mit Endbereich 3a
- 4: Spritze
- 5: Schlauchwandöffnung
- 6: Membran
- 7: schwammartige Umhüllung
- 8: Membranbefestigungsbereich
- 9: Umhüllungbefestigungsbereich
- 10: Befestigungsring
- 11: Gummiüberzug
- 12: Führungsfortsatz
- 13: Endoskop mit Endoskopieschlauch 13a
- 14: Ventil
- 15: Luer(-Lock)-Verbindung

## Patentansprüche

1. Probennahmevorrichtung (1) zum Einführen in den Ösophagus (2) für die Entnahme von Proben zur medizinischen Diagnostik, welche einen Schlauch (3) mit einem in den Ösophagus (2) einführbaren Endbereich (3a) umfasst, sowie einem am Endbereich (3a) befestigten Probennehmer, wobei der Schlauch (3) einen im einführbaren Endbereich (3a) axial geschlossenen Kanal mit einer radialen Schlauchwandöffnung (5) aufweist und entlang eines axialen Abschnittes seines Endbereiches (3a) von einer die Schlauchwandöffnung (5) bedeckenden und aufblasbaren Membran (6) umspannt ist, wobei die Membran (6) in zwei beidseits der Schlauchwandöffnung (5) angeordneten Membranbefestigungsbereichen (8) am Schlauch (3) befestigt und von einer schwammartigen Umhüllung (7) als Probennehmer umgeben ist, **dadurch gekennzeichnet, dass** die schwammartige Umhüllung (7) in zwei beidseits der Schlauchwandöffnung (5) angeordneten Umhüllungsbefestigungsbereichen (9) am Schlauch (3) befestigt ist, und die Umhüllungsbefestigungsbereiche (9) in axialer Richtung weiter voneinander entfernt sind als die Membranbefestigungsbereiche (8).

2. Probennahmevorrichtung (1) nach Anspruch 1, **dadurch gekennzeichnet, dass** die schwammartige Umhüllung (7) in ihren Umhüllungsbefestigungsbereichen (9) mit jeweils zumindest einem Befestigungsring (10) am Schlauch (3) befestigt ist.

3. Probennahmevorrichtung (1) nach Anspruch 2, **dadurch gekennzeichnet, dass** die Umhüllungsbefestigungsbereiche (9) von einem Gummiüberzug umschlossen sind.

4. Probennahmevorrichtung (1) nach einem der Ansprüche 1 bis 3, **dadurch gekennzeichnet, dass** der Schlauch (3) an seinem einführbaren Endbereich (3a) mit einem konisch zulaufenden Führungsfortsatz (12) axial geschlossen ist, welcher das Ende des Schlauches (3) bildet.

5. Probennahmevorrichtung (1) nach einem der Ansprüche 1 bis 4, **dadurch gekennzeichnet, dass** der Schlauch (3) zweilumig ausgeführt ist und in einem der beiden Kanäle ein Versteifungsdraht verläuft.

## Claims

1. A sampling device (1) for inserting into the oesophagus (2) for removing samples for medical diagnostics, which comprises a hose (3) having an end region (3a) that can be inserted into the oesophagus (2), and a sampler fastened to the end region (3a), wherein the hose (3) has an axially closed channel in the insertable end region (3a) having a radial hose wall opening (5) and is encompassed along an axial section of the end region (3a) thereof by an inflatable membrane (6) covering the hose wall opening (5), wherein the membrane (6) is fastened on the hose (3) in two membrane fastening regions (8) arranged on both sides of the hose wall opening (5) and is enclosed by a spongy enclosure (7) as a sampler, **characterized in that** the spongy enclosure (7) is fastened to the hose (3) in two enclosure fastening regions (9) arranged on both sides of the hose wall opening (5) and the enclosure fastening regions (9) are further spaced apart from one another in the axial direction than the membrane fastening regions (8).

2. The sampling device (1) according to claim 1, **characterized in that** the spongy enclosure (7) is fastened to the hose (3) in the enclosure fastening regions (9) thereof by means of at least one fastening ring (10) in each case.

3. The sampling device (1) according to claim 2, **characterized in that** the enclosure fastening regions (9) are surrounded by a rubber coating.

4. The sampling device (1) according to one of claims 1 to 3, **characterized in that** the hose (3) is axially closed at its insertable end region (3a) by a conically tapering guide extension (12) which forms the end of the hose (3).

5. The sampling device (1) according to one of claims 1 to 4, **characterized in that** the hose (3) is designed to be double-lumen and a stiffening wire runs in one of the two channels.

## Revendications

1. Dispositif pour le prélèvement d'échantillons (1) à insérer dans l'oesophage (2) afin de prélever des échantillons en vue de diagnostics médicaux, comprenant un tuyau flexible (3) avec une partie d'extrémité (3a) qui peut être insérée dans l'oesophage (2) et avec un échantillonneur fixé à la partie d'extrémité (3a), dans lequel le tuyau flexible (3) présente dans la partie d'extrémité (3a) insérable un canal fermé dans le sens axial avec une ouverture radiale (5) dans la paroi du tuyau flexible et est entouré le long d'une section axiale de sa partie d'extrémité (3a) par une membrane (6) gonflable couvrant l'ouverture (5) dans la paroi du tuyau flexible, laquelle membrane (6) est fixée au tuyau flexible (3) dans deux zone de fixation de la membrane (8) disposées de part et d'autre de l'ouverture (5) dans la paroi du tuyau flexible et entourée par une enveloppe spongieuse (7) servant de moyen de prélèvement, **caractérisé en ce que** l'enveloppe spongieuse (7) est fixée au tuyau flexible (3) dans deux zones de fixation de l'enveloppe (9) disposées de part et d'autre de l'ouverture (5) dans la paroi du tuyau flexible et les zones de fixation de l'enveloppe (9) sont plus éloignées l'une de l'autre dans le sens axial que les zones de fixation de la membrane (8).

2. Dispositif pour le prélèvement d'échantillons (1) selon la revendication 1, **caractérisé en ce que** l'enveloppe spongieuse (7) est fixée au tuyau flexible (3) dans ses zones de fixation de l'enveloppe (9) avec au moins une bague de fixation (10).

3. Dispositif pour le prélèvement d'échantillons (1) selon la revendication 2, **caractérisé en ce que** les zones de fixation de l'enveloppe (9) sont entourées par un revêtement en caoutchouc.

4. Dispositif pour le prélèvement d'échantillons (1) selon l'une des revendications 1 à 3, **caractérisé en ce que** le tuyau flexible (3) est fermé dans le sens axial dans sa partie d'extrémité (3a) insérable avec une saillie de guidage (12) de forme conique qui forme l'extrémité du tuyau flexible (3).

5. Dispositif pour le prélèvement d'échantillons (1) selon l'une des revendications 1 à 4, **caractérisé en ce que** le tuyau flexible (3) est réalisé avec une double lumière et un fil métallique raidisseur passe dans l'un des deux canaux.
